# EUROPEAN PATENT SPECIFICATION

(11) **EP 3 066 475 B1**
(45) Date of publication and mention of the grant of the patent: **17.07.2019**
(21) Application number: 14783620.9
(22) Date of filing: 10.10.2014
(51) Int. Cl.: G01N 33/68

(54) **BIOMARKERS OF ALZHEIMMER'S DISEASE PROGRESSION**
BIOMARKER DES FORTSCHREITENS VON MORBUS ALZHEIMER
BIOMARQUERS DE LA PROGRESSION DE LA MALADIE D'ALZHEIMER

(30) Priority: 08.11.2013 GB 201319761
(43) Date of publication of application: 14.09.2016
(73) Proprietor: Nordic Bioscience A/S, 2730 Herlev (DK)
(72) Inventor: KARSDAL, Morten, DK-2100 København Ø (DK); HENRIKSEN, Kim, DK-3400 Hillerød (DK)
(74) Representative: Beck Greener LLP
(86) International application number: PCT/EP2014/071766
(87) International publication number: WO 2015/067432

(56) References cited:
- WO-A1-2013/004717
- WO-A2-2011/032155
- WO-A2-2011/123844
- METTE SØRENSEN ET AL: "Utilization of a protease-generated fragment of tau as a biomarker of Alzheimer's disease", ALZHEIMER'S & DEMENTIA, vol. 8, no. 4, 1 July 2012 (2012-07-01), page P240, XP055161182, ISSN: 1552-5260, DOI: 10.1016/j.jalz.2012.05.637 cited in the application
- RISSMAN ROBERT A ET AL: "Caspase-cleavage of tau is an early event in Alzheimer disease tangle pathology.", THE JOURNAL OF CLINICAL INVESTIGATION JUL 2004, vol. 114, no. 1, July 2004 (2004-07), pages 121-130, XP002734348, ISSN: 0021-9738
- BASURTO-ISLAS GUSTAVO ET AL: "Accumulation of aspartic acid421- and glutamic acid391-cleaved tau in neurofibrillary tangles correlates with progression in Alzheimer disease.", JOURNAL OF NEUROPATHOLOGY AND EXPERIMENTAL NEUROLOGY MAY 2008, vol. 67, no. 5, May 2008 (2008-05), pages 470-483, XP009181912, ISSN: 0022-3069

### SEQUENCE LISTING

<110> Nordic Bioscience A/S
<120> Biomarkers of Disease Progression
<130> P18859WO
<150> GB1319761.1
   <151> 2013-11-08
<160> 8
<170> BiSSAP 1.2
<210> 1
   <211> 10
   <212> PRT
   <213> Homo sapiens
<400> 1
   Ser Ser Thr Gly Ser Ile Asp Met Val Asp
   1               5                   10
<210> 2
   <211> 10
   <212> PRT
   <213> Homo sapiens
<400> 2
   Thr Pro Arg Gly Ala Ala Pro Pro Gly Gln
   1               5                   10
<210> 3
   <211> 11
   <212> PRT
   <213> Homo sapiens
<400> 3
   Ser Ser Thr Gly Ser Ile Asp Met Val Asp Ser
   1               5                   10
<210> 4
   <211> 11
   <212> PRT
   <213> Homo sapiens
<400> 4
   Ala Thr Pro Arg Gly Ala Ala Pro Pro Gly Gln
   1               5                   10
<210> 5
   <211> 10
   <212> PRT
   <213> Homo sapiens
<400> 5
   Ser Ser Thr Gly Ser Ile Asp Met Val Asp
   1               5                   10
<210> 6
   <211> 10
   <212> PRT
   <213> Homo sapiens
<400> 6
   Thr Pro Arg Gly Ala Ala Pro Pro Gly Gln
   1               5                   10
<210> 7
   <211> 13
   <212> PRT
   <213> Homo sapiens
<400> 7
   Gly Gly Cys Ser Ser Thr Gly Ser Ile Asp Met Val Asp
   1               5                   10
<210> 8
   <211> 13
   <212> PRT
   <213> Homo sapiens
<400> 8
   Thr Pro Arg Gly Ala Ala Pro Pro Gly Gln Gly Gly Cys
   1               5                   10

## Description

The present invention relates to biomarkers indicating the propensity of a patient to suffer progression of Alzheimer's Disease (AD). Such biomarkers are useful for providing prognostic information to the patient and for selecting candidate patients for clinical trials as well as for evaluating candidate interventions such as medicament treatments for effectiveness in reducing AD progression.

Alzheimer's disease (AD) is estimated to start developing 10-15 years before clinical characteristics become noticeable, a phenomenon which appears to be the primary culprit for the lack of success in developing disease modifying treatments for AD (Cummings, 2011). This has led to an intense search for early fluid-based diagnostic and/or prognostic markers of AD, and cerebrospinal fluid (CSF) biomarkers have provided diagnostic value for AD. However, their application is limited owing to the invasiveness of lumbar puncture (Cummings, 2011). In addition, neuroimaging techniques, such as florbetabir F-18 analysis, provide highly relevant information, but are expensive and require special facilities. Therefore new methods to diagnose the early neurological changes in AD or to forecast the rate of progression of AD with a broader clinical application, such as blood-based methods, are sought.

Potential candidate biomarkers are protein fragments which reflect specific cleavage sites in proteins, and, due to their smaller size, may pass the blood brain barrier (BBB) and thereby be detected in serum (Wang et al., ;Reifert et al., 2011;Karsdal et al., 2010).

The pathological processing of the protein Tau by proteases has generated substantial interest (De Strooper, 2010), as this appears to be a key initiator of neuronal cell death (Reifert et al., 2011;Reifert et al., 2011); (Gamblin et al., 2003;Garcia-Sierra et al., 2008;Goedert et al., 1989;Lasagna-Reeves et al., 2012). Proteolytic cleavage of Tau is mediated by several different proteases, such as caspases and calpains (De Strooper, 2010). Caspase-mediated cleavage of Tau leads to generation of a series of well-described fragments during initiation of neuronal cell death (De Strooper, 2010; Reifert et al., 2011). There are sites in Tau for caspase cleavage, namely residues Asp²²-Asp²⁵, Asp³⁴⁵-Asp³⁴¹ and Asp⁴¹⁸-Asp⁴²¹, of which Asp⁴²¹ is the preferred cleavage site (Hanger and Wray, 2010). The cleavage at Asp⁴²¹ is generated by caspase -3 and -7, and interestingly, the fragments of Tau generated by cleavage at the Asp⁴²¹ site are neurotoxic (Lee et al., 2001;Rissman et al., 2004), and thus measuring this epitope (i.e. fragments having Asp⁴²¹ at their N-terminus) in serum/plasma is of great interest. Polyclonal antibodies which detect the C-terminus fragments of Tau that have been cleaved at Asp⁴²¹ by caspase were also described ((Rissman et al., 2004; Basurto-Islas et al., 2008).

However, several other proteases also appear to play a role in neuronal degeneration even though they mainly have been associated with secretase functions (De Strooper, 2010), and a recent study indicated a relationship between an ADAM10-generated fragment of Tau and cognitive scores (Sorensen et al., 2012), hence highlighting this fragment as a potentially interesting biomarker of AD in combination with the "classical" Tau-C fragment.

WO2013/004717 and Mette et al., 2012 describe a bioassay for the quantification of peptide fragments relevant to neurodegenerative conditions comprising a neo-epitope formed by cleavage of a Tau protein by a secretase (Tau-A). WO2011/ 032155 describes processes and materials for the detection, diagnosis, or determination of the severity of a neurological injury or condition. Biochemical pathways

We have now measured the levels of Tau-C using a newly developed ELISA specifically detecting the terminal Asp⁴²¹ site and the previously published ELISA detecting Tau-A (Sorensen et al., 2012) in serum samples from study H6L-MC-LFAN. We correlated the biomarker levels to ADAS-Cog11 and CDR-SB scores obtained at baseline and at follow-up time points (28, 40, 52, and 64 weeks) to investigate the diagnostic and prognostic potential of these markers.

We have now established that levels of Tau-A and Tau-C are oppositely correlated to future changes in cognitive score markers.
Accordingly, the present invention now provides a method of assay of a sample of body fluid which comprises, in either order:
a) conducting a first immunoassay of the sample to determine a first level of binding therein of an antibody having specificity for a Tau-C C-terminal epitope present at the terminus of the amino acid sequence -SSTGSIDMVD(SEQ ID NO:1), and
b) conducting a second immunoassay of the sample to determine a second level of binding therein of an immunological binding partner having specificity for a Tau-A N-terminal epitope present at the terminus of the amino acid sequence TPRGAAPPGQ- (SEQ ID NO:2), and
c) calculating an index expressing the information content of a ratio between said second level and said first level.

Such an index may be calculated by using a suitably programmed general purpose computer to receive the determined binding levels as inputs and to calculate the index.

Specificity for the terminal epitopes referred to implies that the first immunological binding partner does not bind an extended amino acid sequence -SSTGSIDMVDS (SEQ ID NO:3) or a longer sequence further extended with amino acids continuing the sequence of Tau in the C-terminal direction and that the second immunological binding partner does not bind an extended amino acid sequence ATPRGAAPPGQ- (SEQ ID NO:4) or a longer sequence continuing the sequence of Tau in the N-terminal direction. Preferably, the immunological binding partners do not react with peptides having the terminal sequences -SSTGSIDMVD (SEQ ID NO:5) and TPRGAAPPGQ-(SEQ ID NO:6).

Neither immunological binding partner binds the intact, uncleaved Tau protein.

The immunological binding partners may be monoclonal antibodies or fragments of monoclonal antibodies retaining binding properties, such as Fab, F(ab')₂, Fab', scFv, di-scFv, sdAb, chemically linked F(ab')₂, or BiTE fragments.

Said index may preferably be calculated to express the information content of a ratio (Tau-A'/Tau-C'), wherein Tau-A' is said first level and Tau-C' is said second level, each being expressed as a coefficient of the Normal Reference Mean of the measurement. By this means, variations between assays in the absolute values returned for a particular level of analyte may be compensated such that the results obtained using different assays may readily be compared. The respective coefficients may be calculated in a suitably programmed general purpose computer.

The index may of course be the ratio (Tau-A'/Tau-C').

Generally, the information content of Tau-A/Tau-C (or of Tau-A'/Tau-C') may be preserved in any index in which such a ratio is mathematically manipulated such as by taking a logarithm to any base, such as base 10, of the ratio or by inverting the ratio, or by adding or subtracting the ratio to or from some arbitrary constant or by raising the ratio some chosen power, e.g. squaring it. Many other examples will readily occur to those skilled in the art.

Said sample may preferably be a sample of blood, serum, plasma, CSF, saliva, sputum, tissues, extracts of tissue, or BALF fluid.

The invention includes a method for determining which patients should be enrolled in a clinical trial of a medicament for treatment or prevention of Alzheimer's disease comprising for each candidate patient conducting a method of assay as described above, and selecting for enrolment patients having an index predictive of a change in ADAS-Cog11 of at least three points over 52 weeks. Alternatively, patients are selected having an index predictive of a change in ADAS-Cog11 of at least three points over 40 weeks. Alternatively, patients are selected having an index predictive of a change in ADAS-Cog11 of at least three points over 28 weeks. Further alternatively, patients are selected having an index predictive of a change in ADAS-Cog11 of at least six points over the stated number of weeks.

The invention further includes a method for determining which patients should be enrolled in a clinical trial of a medicament for treatment or prevention of Alzheimer's disease comprising for each candidate patient conducting a method of assay as described above to provide an index expressing the information content of a ratio (Tau-A'/Tau-C'), and selecting for enrolment patients having an index corresponding to a (Tau-A'/Tau-C') ratio of greater than 1.5.

Optionally, one may select for enrolment patients having a said index corresponding to a (Tau-A'/Tau-C') ratio of greater than 2.0 or alternatively of greater than 2.5 or higher.

The invention further includes a method for determining the efficacy of a candidate intervention for treatment or prevention of Alzheimer's disease comprising carrying out an assay as described above on samples obtained from each patient in a first panel of patients and a second panel of patients, wherein said intervention has been applied to said first panel of patients and a comparator intervention has been applied to said second panel of patients , and comparing the values for said index obtained for said first panel of patients with values for said index obtained for said second panel of patients, and determining whether the candidate intervention shows a superior effect in modulating said index values in comparison to the comparator intervention.

The comparator intervention may be a placebo, an absence of treatment, or an alternative candidate or established therapy, whether of a pharmaceutical, dietary, or behavioural nature.
The invention further includes a method for selecting patients for treatment with an intervention for treatment or prevention of Alzheimer's disease comprising conducting a method of assay as described above and selecting patients for treatment who have a resulting index of any value discussed above.

The invention will be further described and illustrated with reference to the following examples in which reference will be made to the accompanying drawings, in which:
Figure 1 shows changes in cognitive scores (ADAS-Cog11 in Panel A and CDR-SB in Panel B) observed in a 100 patient group over a 64 week period.
Figure 2 shows a scatterplot based on the data in Figure 1 showing in Panel A a scatterplot of baseline values of ADAS-Cog11 against CDR-SB, and showing in Panel B a scatterplot of changes in ADAS-Cog11 against changes in CDR-SB at 64 weeks.
Figure 3 shows correlations between Tau-A' levels and change in ADAS-Cog11 with time. Panels: A) Change over 28 weeks, B) Change over 40 weeks, C) Change over 52 weeks, D) Change over 64 weeks.
Figure 4 shows correlations between Tau-C' levels and change in ADAS-Cog11 with time. Panels: A) Change over 28 weeks, B) Change over 40 weeks, C) Change over 52 weeks, D) Change over 64 weeks.
Figure 5 shows correlations between Tau-A'/Tau-C' ratios and change in ADAS-Cog11 with time. Panels: A) change over 28 weeks, B) Change over 40 weeks, C) Change over 52 weeks, D) Change over 64 weeks.
Figure 6 shows correlations between Tau-A' levels and change in CDR-SB with time. Panels: A) change over 28 weeks, B) Change over 40 weeks, C) Change over 52 weeks, D) Change over 64 weeks.
Figure 7 shows correlations between Tau-C' levels and change in CDR-SB with time. Panels: A) Change over 28 weeks, B) Change over 40 weeks, C) Change over 52 weeks, D) Change over 64 weeks.
Figure 8 shows correlations between Tau-A'/Tau-C' ratios and change in CDR-SB with time. Panels: A) Change over 28 weeks, B) Change over 40 weeks, C) Change over 52 weeks, D) Change over 64 weeks.

### EXAMPLES

### MATERIALS & METHODS

### Generation of monoclonal antibodies:

### Tau-C assay

### Reagents and peptides

The synthetic peptides used for monoclonal antibody production and validation were:
Immunogenic peptide: KLH-GGC-SSTGSIDMVD (Keyhole-Limpet-Hemocyanin) (SEQ ID NO:7).
Selection/screening peptide: SSTGSIDMVD
De-selection peptide: SSTGSIDMVDS

### Development of the ELISA

The methods used for monoclonal antibody development were as previously described (Barascuk et al., 2010;Gefter et al., 1977). Briefly, 4-6-week-old Balb/C mice were immunized subcutaneously with 200µl emulsified antigen and 50µg of the immunogenic peptide. Consecutive immunizations were performed at 2-week intervals in Freund's incomplete adjuvant, until stable sera titer levels were reached, and the mice were bled from the 2nd immunization on. At each bleeding, the serum titer was detected and the mouse with highest antiserum titer was selected for fusion. The selected mouse was rested for 1 month followed by intravenous boosting with 50µg of immunogenic peptide in 100µl 0.9% sodium chloride solution 3 days before isolation of the spleen for cell fusion.

### Fusion

The fusion procedure has been previously described (Gefter et al., 1977) and was followed with SP2/0 as myeloma cells. The fusion cells were cloned in 35-mm cell culture dishes by the semi-solid medium method and the dishes were incubated in a CO₂-incubator. Next, clones were plated into 96-well microtiter plates and left for three days, followed by screening of culture supernatants.

### Antibody screening

Supernatants were screened in a competitive ELISA setting. Screening peptides were used as the selection peptides and compared to the elongated peptides. Cell lines specific to the selection peptide and without cross-reactivity to the elongated peptide were selected and the antibodies were purified.

### ELISA methodology

In preliminary experiments, we optimized the reagents, their concentrations and the incubation periods by performing several checkerboard analyses (data not shown).

### ELISA methodology Tau-C

The final Tau-C ELISA was developed as follows: A 96-well ELISA plate pre-coated with streptavidin was further coated with 1.25ng/ml of the synthetic peptide Biotin-SSTGSIDMVD dissolved in PBS-BT buffer at 4°C for 30 min by constant shaking at 300 rpm. The plate was washed five times in washing buffer and 20µl of sample was added, followed by 100µl of peroxidase conjugated anti-human mAb-Tau-C solution (50ng/ml). The plate was incubated for 1 h at 4°C in assay buffer (10 mM PBS, 68mM NaCl, 1% BSA, 0.05% Tween-20, 0.36% Bronidox L5, and 10% Liquid II) during which time it was shaken at 300 rpm.

The plate was again washed five times followed by the addition of 100µl tetramethylbenzidine (TMB) (Kem-En-Tec cat.438OH). The plate was incubated for 15 min in darkness and shaken at 300rpm. In order to cease the reaction, 100µl of stopping solution (95-97% H₂SO₄, Merck Cat. No.: 1.00731) was added and the plate was analyzed in the ELISA reader at 450 nm with 650 nm as the reference.

### Buffers used for the ELISAs

Buffer used for dissolving the coating peptide was composed of the following: (10mM PBS, 68mM NaCl, 1% BSA, 0.05% Tween-20, 0.9% EDTA, 0.36% Bronidox L5, and 10% Liquid II pH=7.4), and reaction stopping buffer composed of 0.1% H₂SO₄.

ELISA-plates used for the assay development were Streptavidin-coated from Roche cat.: 11940279. All ELISA plates were analysed with the ELISA reader from Molecular Devices, SpectraMax M, (CA, USA).

### Standards

A standard curve was performed by serial dilution of the selection peptides. Standard concentrations for Tau-C were 0, 0,97, 1,95, 3,9, 7,8, 15,6, 31,25, 62,5, 125, 250, 500, and 1000ng/ml.

### Samples for testing native reactivity of the antibodies

For assay development and validation, serum and plasma from 15 healthy adult volunteers aged 23 - 45 years and of both genders were used (Bioserve). We also tested serum samples from mice and rats to determine the level of interspecies cross reactivity.

### Animal samples

Tissues including brain, liver, muscle, colon, kidney, lung, aorta, heart, skin and pancreas isolated from 5 six-month-old Sprague Dawley rats and 5 brains from each of either the wildtype or Tg4510 mice were flash-frozen in liquid nitrogen and pulverized using a Bessman pulverizer. The "powder" was transferred to a vial and weighed. Extraction buffer (50mM Tris-HCl, 50mM HEPES, 1mM EDTA, 0.5% sodium deoxycholate, 15% glycerol, protease inhibitor cocktail (Roche cat#05056489001), pH8.3) was added at 1mL buffer/250mg tissue. The lysate was cleared by sonication. After sonication the debris was spun at 4°C / 5 min / 10000rpm and the supernatants were collected and stored at - 80°C until further use.

Protein concentrations were determined using the DC Protein Assay (Biorad).

### In vitro cleavage of tissues

Protease cleavage was performed by mixing 1µg of recombinant Tau or 50 µg of tissue extract and 1 µg of Caspase in Caspase buffer (25mM HEPES, 0,1% CHAPS, 5 mM DTT, 0,5 mM EDTA, 10% glycerol, pH 7.5) and incubating for 6hr, 1day and 2 days. Finally, the cleavage was verified by ELISA analysis.

### PC12 cell experiments

PC12 cells were obtained from ATCC and cultured in flasks or plates coated with 0.01% collagen solution. The cells were cultured in RPMI-1640 + 10% horse serum + 5% FCS + 1% P/S + 200mM Glutamate with medium exchange every 2^{nd}-3^{rd} day. To differentiate them into neurons the cells were seeded in pre-coated plates at the density of 10000 cell/cm² and cultured for 8 days in RPMI-1640 + 1% horse serum + 1% P/S + 200mM Glutamate with 50ng/mL human NGF (R&D Systems). On day 8 apoptosis was induced using Aβ as described by (Reifert et al., 2011), and lysates were generated using RIPA+++ buffer on the indicated time points.

### Western Blotting

20µg of each lysate was loaded onto an SDS-PAGE gel, and the gel was run, followed by transfer of the proteins to nitrocellulose membranes. Ponceau Red staining was then used to verify equal protein loading on the membranes. Total Tau protein (MAB3420, Millipore) was detected by incubation with the primary antibodies diluted to 100ng/mL in TBS-T containing skim milk powder (Henriksen et al., 2004). A secondary antibody recognizing mouse IgG conjugated to horse-radish peroxidase was then added, and finally the blot was visualized using enhanced chemiluminescence as previously described (Henriksen et al., 2004).

### RESULTS

### Selection of clone and characteristics of the assay

Immunization of mice with the identified sequence generated numerous clones which then were screened for their ability to produce an antibody (NB361) recognizing the sequence SSTGSIDMVD generated by Caspase 3 cleavage of Tau.

The antibody was specific for the cleavage site, as extension of the sequence by one amino acid, to create an elongated peptide, led to loss of reactivity and only very modest cross-reactivity to the elongated sequence. Based on the standard curve the lower limit of detection (LDL) was determined to be 1ng/mL.

### Characterization of the assay using in vitro generated material

As the epitope was identified from Caspase-cleaved Tau, we investigated whether *in vitro* cleavage of Tau using Caspase resulted in the generation of a signal in the ELISA. Only the addition of cleaved Tau, not intact Tau, led to the generation of a signal, further confirming the specificity of the assay. These data were further strengthened by comparing caspase cleaved rat brain to non-cleaved and again here only the cleaved samples gave a signal.

### Brain extracts from tg4510 mice indicate pathological relevance of the marker

The Tg4510 mice are known to show a Tauopathy with onset from around two months, and when measuring brain extracts from Tg4510 mouse brains had around 2-3 folds higher levels of Tau-C than their corresponding wt controls both at 1½, 2½ and a trends towards a clearer separation at 9 months.

### Aβ-mediated apoptosis in neurons derived from PC12 cells leads to production of Tau-C

The Tau-C fragment has been indicated to be produced by neurons upon induction of apoptosis, and to assess whether this could be measured using the ELISA, we used the NGF-induced PC12 neuronal cells. When culturing the PC12 cells for 8 days in the presence of NGF a clear induction of neurogenesis, and accordingly induction of total Tau expression was observed as expected(Greene, 1978).

Addition of Aβ to neurons has been shown to induced neuronal apoptosis and Tau fragmentation (Reifert et al., 2011), and addition of high concentrations (8.6µM) was found to lead to fragmentation of Tau already after 5 hours, and after 24 hours no Tau was seen. At a lower dose (0.86 µM) of Aβ no fragmentation was observed. Interestingly, when measuring the cell lysates from this experiment in the Tau-C ELISA a marked increase was observed in the Aβ high conditions at 5 and 24 hours, while only background levels were observed in the other conditions. These data, while preliminary, indicate that the Tau-C fragment indeed is produced in conjunction with neuronal death.

### The Tau-C ELISA detects degradation fragments of Tau in serum from humans, rats and mice, and it is technically robust

Dilution recovery tests of human serum and plasma, and rat and mouse serum, showed that these samples could all be diluted and provide linear recoveries when measuring at the right dilutions. For human sera and plasma, the dilution range was between 1+1 and 1+3, for rat serum it was between 1+1 and 1+3 and finally in mouse serum the accepted dilution range was 1+1and 1+3. The intra-assay coefficient of variation, determined by measuring 3 individual serum samples 21 times each, was 5.8%. The inter-assay CV%, determined by measuring 8 different samples in duplicates in 11 different runs over a total of 5 individual days, was 12.6%. Finally, a series of three serum samples exposed to 5 consecutive freeze-thaw cycles did not show a loss of reactivity.

### Tau-A assay

### Reagents and peptides

The synthetic peptides used for monoclonal antibody production and validation were:
(a) immunogenic peptide: TPRGAAPPGQ-GGC-KLH (SEQ ID NO:8) (Keyhole-Limpet-Hemocyanin),
(b) screening peptide TPRGAAPPGQ (SEQ ID NO:2),
(c) de-selection peptide **A**TPRGAAPPGQ (SEQ ID NO:4) which has been elongated with one amino acid in the N-terminus.

### Buffers used for the ELISA

Buffer used for dissolving the coating peptide was composed of the following: (150mM Trizma, 1% BSA, 0.05% Tween-20, 0,36% Bronidox L5, pH 8.0 (Tris-BTB), and reaction stopping buffer composed of 0,1% H₂SO₄.

ELISA-plates used for the assay development were Streptavidin-coated from Roche cat.: 11940279. All ELISA plates were analysed with the ELISA reader from Molecular Devices, SpectraMax M, (CA, USA).

### Development of an ELISA

Methods for monoclonal antibody development are previously described (Barascuk et al., 2010); Gefter, 1977 1917).

Briefly, 4-6-week-old Balb/C mice were immunized subcutaneously with 200µl emulsified antigen and 50µg TPRGAAPPGQ-GGC-KLH (SEQ ID NO:2-KLH). Consecutive immunizations were performed at 2-week intervals in Freund's incomplete adjuvant, until stable sera titre levels were reached, and the mice were bled from the 2nd immunization on. At each bleeding, the serum titre was detected and the mouse with highest antiserum titre was selected for fusion. The selected mouse was rested for 1 month followed by intravenous boosting with 50µg TPRGAAPPGQ-GGC-KLH (SEQ ID NO:2-KLH) in 100µl 0.9% Sodium Chloride solution 3 days before isolation of the spleen for cell fusion.

### Fusion

The fusion procedure previously described (Gefter et al., 1977) was followed with SP2/0 as myeloma cells. The fusion cells were cloned in 35-mm cell culture dishes by the semi-solid medium method and the dishes were incubated in a CO₂-incubator. Next, clones were plated into sixteen 96-well microtiter plates and left for three days, followed by screening of culture supernatants.

### Antibody screening

Supernatants were screened in a competitive ELISA setting. Peptide TPRGAAPPGQ (SEQ ID NO:2) was used as the selection peptide and the **A**TPRGAAPPGQ (SEQ ID NO:4) as the elongated peptide. Cell lines specific to selection peptide and without cross-reactivity to the elongated peptide were selected and the antibodies were purified.

### Tau-A ELISA methodology

In preliminary experiments, we optimized the reagents, their concentrations and the incubation periods by performing several checkerboard analyses. The Tau-A ELISA was developed as follows: A 96-well ELISA plate pre-coated with streptavidin was further coated with 6ng/ml of the synthetic peptide TPRGAAPPGQ-Biotin (SEQ ID NO:2-Biotin) dissolved in Tris-BTB buffer at 20°C for 30 min by constant shaking at 300 rpm. The plate was washed five times in washing buffer and 20µl of sample was added, followed by 100µl of peroxidase conjugated anti-human mAb-Tau-A solution (50ng/ml). The plate was incubated for 1 h at 20°C in 100 mM Tris-BTB buffer during which time it was shaken at 300 rpm.

The plate was again washed five times followed by addition of 100µl tetramethylbenzidine (TMB) (Kem-En-Tec cat.438OH). The plate was incubated for 15 min in darkness and shaken at 300rpm. In order to cease the reaction, 100µl of stopping solution (95-97% H2SO4, Merck Cat. No.: 1.00731) was added and the plate was analysed in the ELISA reader at 450 nm with 650 nm as the reference.

### Standards

A standard curve was performed by serial dilution of TPRGAAPPGQ-biotin (SEQ ID NO:2-biotin). Standard concentrations were 0, 0.782, 1.5625, 3.125, 6.25, 12.5, 25, 50, and 100ng/ml.

### Samples for testing native reactivity of the antibodies

During assay development and validation serum from healthy adult subjects of different age and gender were used. Serum samples are obtained from young healthy volunteers 23-45 years of age. We also tested serum samples from different species including mouse and rat to determine the level of interspecies cross reactivity. Finally, we also tested cerebrospinal Fluid (CSF) and plasma samples.

### Animal samples

Flash frozen brains from 5 wildtype and 5 Tg4510 Tauopathy mice aged 5 months (de Calignon et al., 2009) were extracted according to the following protocol: The tissue was pulverized using a Bessman pulverizer and weighed. The tissue was extracted using 250mg tissue/mL extraction buffer (50mM Tris-HCl, 50mM HEPES, 15% glycerol, 1mM EDTA, 0.5% sodium deoxycholate, Roche protease inhibitor (cat# 05 056 489001), final pH8.3). The lysate was cleared by sonication, and the supernatants were collected after centrifugation at 4°C / 5 min / 10000rpm. Protein concentration was determined using the DC Protein Assay from BioRad.

### Western Blotting

100µg of each extract was loaded onto an SDS-PAGE gel. The gel was run and the samples were transferred to nitrocellulose membranes as described by (Henriksen et al., 2004). The levels of Tau-A fragments and total Tau protein were detected by incubation with the primary antibodies diluted to 100ng/mL in TBS-T containing skim milk powder (Henriksen et al., 2004). A secondary antibody recognizing mouse IgG conjugated to horse-radish peroxidase was then used, and finally the blot was visualized using enhanced chemiluminescence as previously described (Henriksen et al., 2004).

### Human samples

Two sets of human samples were used. One set (51 samples) was a collection of serum samples from an osteoarthritis study, this study contained participants with ages from 18-75, as well as Body Mass Index data (Bay-Jensen et al., 2011).

The second set of samples was paired serum and CSF samples from patients with severe Alzheimer's disease. These samples contained a subset of samples collected within the same individuals at baseline and follow-up (18 months later).

### Statistical analysis

For assay validation, optical density was fitted against analyte concentration applying a four-parameter logistic regression to the calibration curve. Average, standard deviations, percentage coefficient of variation (%CV), and differences from theoretical values were calculated for all standards and samples. Quantitative data were analysed using GraphPad Prism 5 (GraphPad Software, San Diego, CA, USA). Significant differences between means were determined using the Student's two-tailed unpaired t-test, not assuming Gaussian distribution. Correlations between serum Tau values and the rest of the variables studied were analysed using Linear Regression. Data was expressed as mean ± standard error of the mean and differences were considered significant at a p level of 0.05 or lower.

### RESULTS

### ELISA technical specifications

The antibody with best native reactivity, affinity and stability in the assay was chosen from the antibody-producing clones generated after the fusion of spleen- and myeloma cells. The clone chosen for antibody purification and the subsequent development of the ELISA was NB191-3C4, raised against TPRGAAPPGQ (SEQ ID NO:2).

### Standard curve and recovery

A typical standard curve was obtained based on peptide concentrations of 0, 0.782, 1.5625, 3.125, 6.25, 12.5, 25, 50, and 100ng/mL. In addition the specificity of the antibody was shown by comparison to the same concentrations of the elongated peptide which gave no reaction at all.

Determination of the linearity or recovery by dilution in different samples resulted in following. The average determined recoveries back-calculated from samples diluted 1+1, 1+2, 1+3, 1+4, 1+5, 1+6 and 1+7 to undiluted sample were close to 100% and within the recommended ±10% (data not shown).

### Calculation of biomarker results

The value a of normal reference mean (NRM) was established for both the Tau-A and the Tau-C assays as the mean value of each biomarker measured on 15 samples from healthy age and sex-matched control individuals ('normals').

All biomarker data from the AD subjects are presented herein as a ratio between the absolute value and the NRM values to take into account differences between different cohorts.
Hence Tau-A' was calculated as Tau-A/NRM_{Tau-A}
Hence Tau-C' was calculated as Tau-C/NRM_{Tau-C}
Thus, the Tau-A' and Tau-C' present the values of Tau-A and Tau-C as coefficients of the respective NRM.

### Example 1: Diagnostic and prognostic value of the assays

### Subjects:

Baseline serum samples were obtained from Early Alzheimer's patients (n=100) for which serum samples were banked at baseline), with ADAS-Cog11, CDR-SB and MMSE scores assessed at baseline (n=100), and ADAS-Cog11 and CDR-SB assessed at follow-up time points of 28, 40, 52 and 64 weeks in a subset of the cohort (placebo arm)(n=35).

The inclusion criteria were: 55 Years and older, Meets criteria for mild to moderate AD with Mini-Mental State Examination score of 16 through 26 at visit 1. Modified Hachinski Ischemia Scale score of less than or equal to 4. Geriatric Depression Scale score of less than or equal to 6. A magnetic resonance imaging (MRI) or computerized tomography (CT) scan in the last 2 years with no findings inconsistent with a diagnosis of Alzheimer's disease. If female must be without menstruation for at least 12 consecutive months or have had both ovaries removed. The exclusion criteria were: Is not capable of swallowing whole oral medication. Has serious or unstable illnesses. Does not have a reliable caregiver. Chronic alcohol or drug abuse within the past 5 years. Has ever had active vaccination for AD

### Endpoints:

Correlations to baseline ADAS-Cog11 and CDR-SB. Correlations to change in ADAS-Cog11 and/or CDR-SB over time

### Statistical analysis

All data were checked for normality using the Shapiro-Wilks test. Data that are not normally distributed (e.g., p < 0.05) were log-transformed to normality. The correlations between plasma Tau levels and the endpoints were analyzed using linear regression. An MMRM-model was used to compare change in cognitive scores to Tau-A', Tau-C' or the Tau-A'/Tau-C' ratio, while adjusting for age and onset and baseline cognitive score.

### RESULTS

### Patient characteristics:

The characteristics of the subpopulation of studies (NCT00762411 and NCT00594568) are shown in Table 1.

**Table 1: Baseline characteristics of the subjects.**

| **Age in years (SD)** | **M/F** | **Duration of Disease (years)** | **ADAS-Cog11** | **CDR-SB** | **MMSE stratification (mild/moderate)** |
|---|---|---|---|---|---|
| 71.6 (7.7) | 47=1/52=2 | 5.4 (2.6) | 23.9 (9.6) | 4.8 (3.2) | 68/32 |

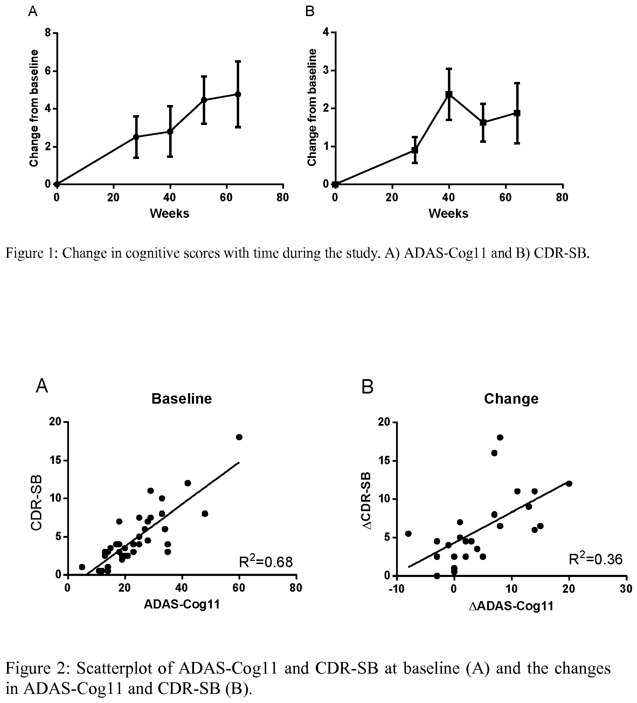

As intended the population of 100 patients are in the mild to moderate AD category, and thus represent a classical target population for a drug intervention study (Cummings, 2011), and hence also represent a population which is of great interest for biomarker studies. Figure 1 shows the change in cognitive scores ADAS-Cog11 and CDR-SB over 64 weeks, whilst Figure 2 shows the correlation between the two different cognitive scores at baseline and between their changes over the 64 weeks. In terms of cognitive change during the study the change in ADAS-Cog11 over the 64 week study period was 4.7+/-1.7 with a continuous increase seen at all time points, clearly underscoring that progression took place. For CDR-SB the increase was 1.9+/-0.8. However, the variation in the change of the cognitive scores was substantial as expected for a cognitive assessment.

### Baseline biomarker correlations to cognitive scores.

To investigate the clinical relevance of the Tau fragments in serum samples, we correlated the Tau-A' and Tau-C' values¹ obtained to baseline cognitive assessments using both ADAS-Cog11 and CDR-SB, and we found no relationship between baseline biomarker values and cognitive scores (data not shown).
¹ Tau-A and Tau-C relative to their respective normal reference means as described above

### Biomarker correlation to change in ADAS-Cog11 score.

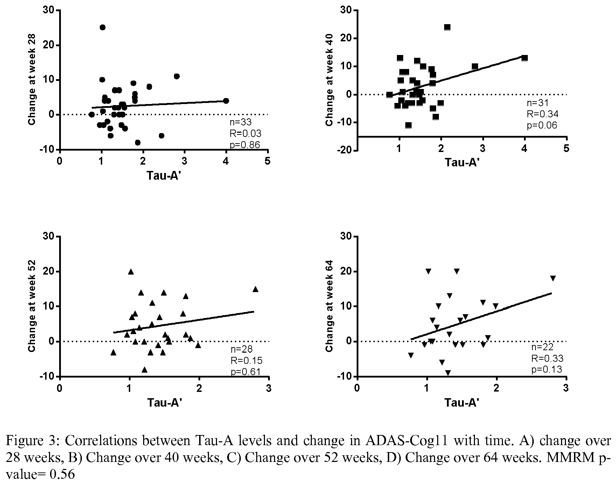

To investigate the relationship between baseline biomarker levels and change in cognitive function a series of linear regressions were performed. In figure 3A-D the correlation between Tau-A levels and ADAS-Cog11 at the different follow-up time points is seen, and a trend between baseline Tau-A' levels and change in ADAS-Cog11 is apparent at the three late time points, but not the early time point (28 weeks).

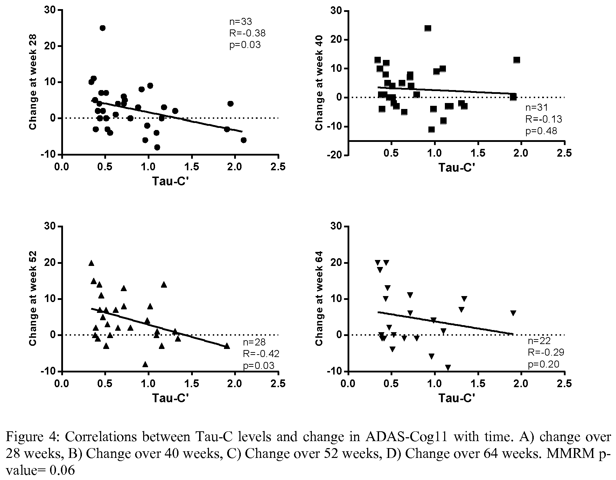

With respect to Tau-C' levels measured at baseline, we observed trends or statistically significant inverse correlations to change in ADAS-Cog11 at the different follow-up time points of 28, 40, 52 and 64 weeks (Figure 4A-D), indicating some relevance for this marker as an indicator of disease progression.

The relationships between Tau-A' and change in ADAS-Cog11 and Tau-C' and change in ADAS-Cog11 were opposite, clearly indicating that Tau-A' and Tau-C' represent independent pathological aspects of Alzheimer's disease.

With this finding in mind we combined the two markers in a ratio to investigate whether the combination of the markers could help avoid some of the intrinsic variation in the cognitive score dataset (CV% on change in ADAS-Cog11 is 137%). With respect to baseline cognitive scores no correlations were observed (data not shown).

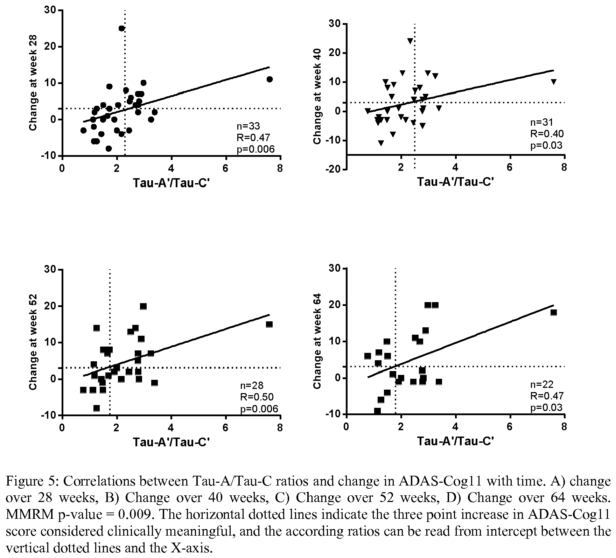

As seen in Figure 5, a combination of the two serum biomarkers led to stronger and statistically significant correlations when looking at all the individual follow-up time points, and even when removing the most extreme points from the correlations, the correlations were still statistically significant (data not shown).

### Biomarker correlation to change in CDR-SB score.

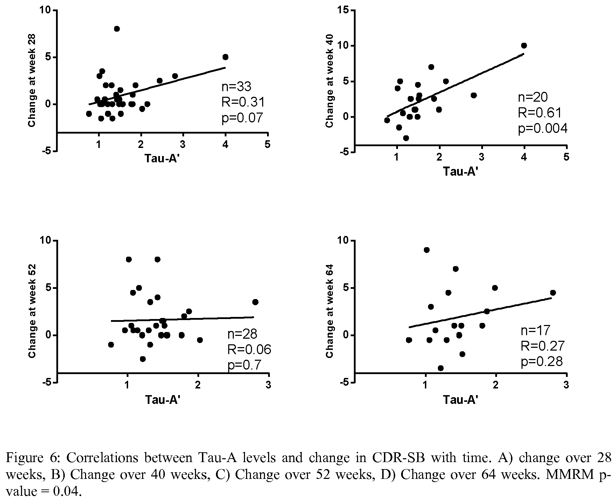

We next investigated the relationship between baseline biomarker levels and change in clinical dementia rating - sum of boxes, again using a series of linear regressions. In Figure 6, panels A-D the correlations between Tau-A' levels and CDR-SB at the different follow-up time points are shown, and an overall trend towards a positive relationship between baseline Tau-A levels and change in CDR-SB is apparent, and even reaches statistical significance at week 40.

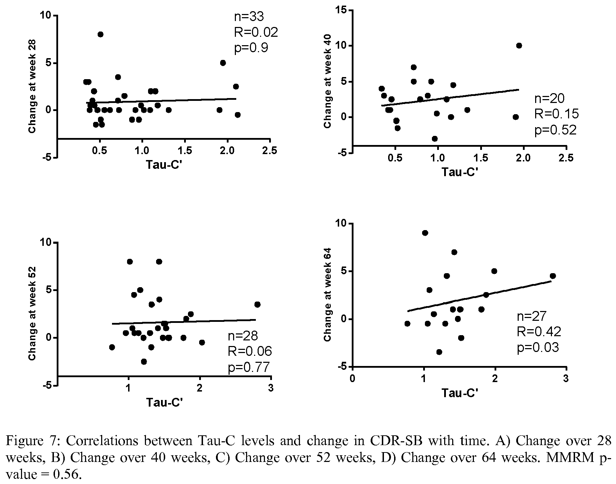

With respect to Tau-C' levels, we observed a slightly more variable picture with the overall trend towards a positive relation between Tau-C' and change in CDR-SB (Figure 7, panels A-D). However, as seen in Figure 7, panel C the large variation in the change in CDR-SB from time point to time point is an issue.

The relationships between Tau-A' and change in CDR-SB and Tau-C' and change in ADAS- CDR-SB were unrelated, which, as for ADAS-Cog11, indicates that Tau-A' and Tau-C' represent independent pathological aspects of Alzheimer's disease. Therefore, we again combined the two markers in a ratio to see if this could help reduce some of the variation. With respect to baseline cognitive scores no correlations were observed (data not shown).

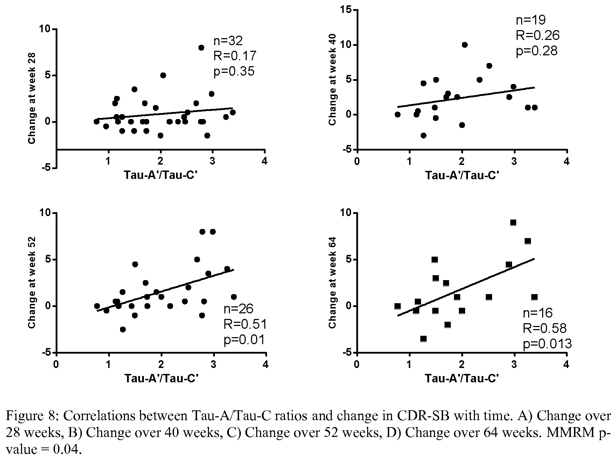

As seen in Figure 8, a combination of the two serum biomarkers showed a continuous improvement of the correlation of the serum marker ratio to change in CDR-SB.

As the linear regression analyses were constructed independently of baseline parameters and only show snapshots of the data, we performed an MMRM-analysis (Mixed-Effect Model Repeated Measure), and in this model we adjusted for baseline cognitive score and age at onset, and we found a highly interesting relationship between Tau-C and change in ADAS-Cog11 (p=0.06), and a stronger relation for the Tau-A/Tau-C ratio (p=0.009) (see Table 2).

**Table 2: MMRM model p-values; VISID 11, 12, 13, 14. Model change=baseline + ageyr + log10(Tau)**

| **Score** | **N** | **Tau A** | **Tau C1** | **Tau A/C** |
|---|---|---|---|---|
| ADAS-Cog11 | 35 | 0.56 | 0.06 | 0.009 |
| CDR-SB | 34 | 0.04 | 0.56 | 0.04 |

For CDR-SB, we observed that Tau-A' was significantly related to change (p<0.04), while Tau-C' or the combination did not alter this output. These data really strengthen the idea that Tau-fragments present in serum can provide the rate of progression for AD, and also illustrate that the different Tau-fragments are related to different pathological aspects.

### DISCUSSION

Potential serum and/or plasma-based markers for AD have been investigated extensively. While many have been studied, none of these have been able to indicate the rate of disease progression, a parameter, which is of crucial importance for the development of disease modifying treatments of AD (Han et al., 2011;Mielke et al., 2011;Ray et al., 2007;Henriksen et al., 2013).

In relation to disease progression of Alzheimer's disease, several studies have indicated that Tau protein is proteolytically processed during neuronal death, and that one of the proteases involved in this process is caspase-3 (Hanger and Wray, 2010;Lee et al., 2001;Rissman et al., 2004;de et al., 2010;Gamblin et al., 2003;Park et al., 2007), and hence monitoring caspase fragments of Tau, such as Tau-C, is of substantial interest in Alzheimer's disease. Secondly, a recent study indicated the pathological relevance of an ADAM10-generated fragment of Tau (Tau-A) as a serum marker of cognitive function (Sorensen et al., 2012).

Therefore, we applied an ELISA measuring Tau-C' in serum, as well as the previously described Tau-A' assay to serum samples collected at baseline of a phase III clinical trial, and correlated the levels of these biomarkers to cognitive score as well as changes in cognitive score over time.

We did not find any relationship to baseline ADAS-Cog11 or CDR-SB for either Tau-A', Tau-C' or the ratio between these, a finding which indicates that these markers do not have diagnostic potential in this setting, i.e. early AD patients. In contrast, an indication towards a positive relation between serum Tau-A' and change in ADAS-Cog11 and a negative relation between serum Tau-C' and change in ADAS-Cog11 were observed. These data support that measurement of serum Tau-fragments, representing various steps of the pathology, can provide the rate of disease progression. Interestingly, combining the two serum biomarkers in a ratio provided a better correlation to change in ADAS-Cog11 and resulted in statistical significance at all the investigated time points. Importantly, our MMRM analysis confirmed these findings and underscored the relationship between Tau-C' as well as the ratio between Tau-A' and Tau-C' and ADAS-Cog11 when adjusting for confounders, such as age at disease onset and baseline cognitive score. The combination of these two biomarkers provides a way for reducing some of the variation associated with a cognitive assessment.

When we correlated the levels of Tau-A', Tau-C' and the ratio between these two to changes in CDR-SB, we found that Tau-A' correlated well with change in CDR-SB; while there did not appear to additional information in measuring Tau-C' or the ratio, data which were also confirmed using the MMRM model.

To clarify whether the difference between Tau-A' and Tau-C' and their relations to CDR-SB and ADAS-Cog11 respectively, could be biologically meaningful, we correlated the cognitive scores, and the change in cognitive scores respectively and here we found that baseline ADAS-Cog11 correlated to baseline CDR-SB with a correlation coefficient of R²=0.68; however, when correlating the change in ADAS-Cog11 to CDR-SB the correlation had an R² of 0.36. These data indicate that the changes in the cognitive scores are partially due to different aspects of disease, as illustrated by the differences in how these scores are obtained (O'Bryant et al., 2010;Rosen et al., 1984;Hughes et al., 1982).

In relation to classical biomarkers of AD, such as CSF-Tau, CSF-phospho-Tau, Amyloid beta (1-40 and 1-42) in both CSF and plasma we did not observe any correlation of the TauA, TauC or their ratio to these at baseline (data not shown), data which indicate that the two Tau-fragments provide information not directly related to already existing fluid biomarkers. Existing biomarkers mainly are relevant for diagnosis of AD, as well as segregation of these from other types of dementia, but do not have the capacity to predict disease progression (Cummings, 2011;Frank and Hargreaves, 2003;Hampel et al., *2010*;*Zetterberg*, 2008). There are some indications that plasma Abeta levels can predict cognitive decline in healthy elderly (Yaffe et al., 2011); however, recent publications on plasma Abeta have indicated that more studies are needed before the full understanding is present (Toledo et al., 2013).

Importantly, these data indicate that the ratio between Tau-A and Tau-C, optionally calculated as the ratio between Tau-A' and Tau-C', provides a tool for measuring the rate of disease progression in early AD, and thereby will be useful for identifying subjects to be enrolled in clinical trials of disease modifying drugs.

In this specification, unless expressly otherwise indicated, the word 'or' is used in the sense of an operator that returns a true value when either or both of the stated conditions is met, as opposed to the operator 'exclusive or' which requires that only one of the conditions is met. The word 'comprising' is used in the sense of 'including' rather than in to mean 'consisting of'.

### References

1. Barascuk, N., S.S.Veidal, L.Larsen, D.V.Larsen, M.R.Larsen, J.Wang, Q.Zheng, R.Xing, Y.Cao, L.M.Rasmussen, and M.A.Karsdal. 2010. A novel assay for extracellular matrix remodeling associated with liver fibrosis: An enzyme-linked immunosorbent assay (ELISA) for a MMP-9 proteolytically revealed neo-epitope of type III collagen. Clin. Biochem. 43:899-904.
2. Bay-Jensen, A.C., Q.Liu, I.Byrjalsen, Y.Li, J.Wang, C.Pedersen, D.J.Leeming, E.B.Dam, Q.Zheng, P.Qvist, and M.A.Karsdal. 2011. Enzyme-linked immunosorbent assay (ELISAs) for metalloproteinase derived type II collagen neoepitope, CIIM--increased serum CIIM in subjects with severe radiographic osteoarthritis. Clin. Biochem. 44:423-429.
3. Cummings, J.L. 2011. Biomarkers in Alzheimer's disease drug development. Alzheimers. Dement. 7:e13-e44.
4. de Calignon, A., L.M.Fox, R.Pitstick, G.A.Carlson, B.J.Bacskai, T.L.Spires-Jones, and B.T.Hyman. 2010. Caspase activation precedes and leads to tangles. Nature 464:1201-1204.
5. de Calignon, A., T.L.Spires-Jones, R.Pitstick, G.A.Carlson, and B.T.Hyman. 2009. Tangle-bearing neurons survive despite disruption of membrane integrity in a mouse model of tauopathy. J. Neuropathol. Exp. Neurol. 68:757-761.
6. De Strooper, B. 2010. Proteases and proteolysis in Alzheimer disease: a multifactorial view on the disease process. Physiol Rev. 90:465-494.
7. Frank, R. and R.Hargreaves. 2003. Clinical biomarkers in drug discovery and development. Nat. Rev. Drug Discov. 2:566-580.
8. Gamblin, T.C., F.Chen, A.Zambrano, A.Abraha, S.Lagalwar, A.L.Guillozet, M.Lu, Y.Fu, F.Garcia-Sierra, N.LaPointe, R.Miller, R.W.Berry, L.I.Binder, and V.L.Cryns. 2003. Caspase cleavage of tau: linking amyloid and neurofibrillary tangles in Alzheimer's disease. Proc. Natl. Acad. Sci. U. S. A 100:10032-10037.
9. Garcia-Sierra, F., S.Mondragon-Rodriguez, and G.Basurto-Islas. 2008. Truncation of tau protein and its pathological significance in Alzheimer's disease. J. Alzheimers. Dis. 14:401-409.
10. Gefter, M.L., D.H.Margulies, and M.D.Scharff. 1977. A simple method for polyethylene glycol-promoted hybridization of mouse myeloma cells. Somatic. Cell Genet. 3:231-236.
11. Goedert, M., M.G.Spillantini, R.Jakes, D.Rutherford, and R.A.Crowther. 1989. Multiple isoforms of human microtubule-associated protein tau: sequences and localization in neurofibrillary tangles of Alzheimer's disease. Neuron 3:519-526.
12. Greene, L.A. 1978. Nerve growth factor prevents the death and stimulates the neuronal differentiation of clonal PC12 pheochromocytoma cells in serum-free medium. J. Cell Biol. 78:747-755.
13. Hampel, H., R.Frank, K.Broich, S.J.Teipel, R.G.Katz, J.Hardy, K.Herholz, A.L.Bokde, F.Jessen, Y.C.Hoessler, W.R.Sanhai, H.Zetterberg, J.Woodcock, and K.Blennow. 2010. Biomarkers for Alzheimer's disease: academic, industry and regulatory perspectives. Nat. Rev. Drug Discov. 9:560-574.
14. Han, X., S.Rozen, S.H.Boyle, C.Hellegers, H.Cheng, J.R.Burke, K.A.Welsh-Bohmer, P.M.Doraiswamy, and R.Kaddurah-Daouk. 2011. Metabolomics in early Alzheimer's disease: identification of altered plasma sphingolipidome using shotgun lipidomics. PLoS. One. 6:e21643.
15. Hanger, D.P. and S.Wray. 2010. Tau cleavage and tau aggregation in neurodegenerative disease. Biochem. Soc. Trans. 38:1016-1020.
16. Henriksen, K., J.Gram, S.Schaller, B.H.Dahl, M.H.Dziegiel, J.Bollerslev, and M.A.Karsdal. 2004. Characterization of osteoclasts from patients harboring a G215R mutation in ClC-7 causing autosomal dominant osteopetrosis type II. Am J Pathol 164:1537-1545.
17. Henriksen,K., S.E.O'Bryant, H.Hampel, J.Q.Trojanowski, T.J.Montine, A.Jeromin, K.Blennow, A.Lonneborg, T.Wyss-Coray, H.Soares, C.Bazenet, M.Sjogren, W.Hu, S.Lovestone, M.A.Karsdal, and M.W.Weiner. The future of blood-based biomarkers for Alzheimer's disease. 2013 [Epub ahead of print]
18. Hughes, C.P., L.Berg, W.L.Danziger, L.A.Coben, and R.L.Martin. 1982. A new clinical scale for the staging of dementia. Br. J. Psychiatry 140:566-572.
19. Karsdal, M.A., K.Henriksen, D.J.Leeming, T.Woodworth, E.Vassiliadis, and A.C.Bay-Jensen. 2010. Novel combinations of Post-Translational Modification (PTM) neo-epitopes provide tissue-specific biochemical markers-are they the cause or the consequence of the disease? Clin. Biochem. 43:793-804.
20. Lasagna-Reeves, C.A., D.L.Castillo-Carranza, U.Sengupta, J.Sarmiento, J.Troncoso, G.R.Jackson, and R.Kayed. 2012. Identification of oligomers at early stages of tau aggregation in Alzheimer's disease. FASEB J.
21. Lee, S.E., W.J.Chung, H.B.Kwak, C.H.Chung, K.B.Kwack, Z.H.Lee, and H.H.Kim. 2001. Tumor necrosis factor-alpha supports the survival of osteoclasts through the activation of Akt and ERK. J Biol Chem 276:49343-49349.
22. Mielke, M.M., N.J.Haughey, V.V.Bandaru, D.D.Weinberg, E.Darby, N.Zaidi, V.Pavlik, R.S.Doody, and C.G.Lyketsos. 2011. Plasma Sphingomyelins are Associated with Cognitive Progression in Alzheimer's Disease. J. Alzheimers. Dis.
23. O'Bryant, S.E., S.C.Waring, V.Hobson, J.R.Hall, C.B.Moore, T.Bottiglieri, P.Massman, and R.az-Arrastia. 2010. Decreased C-reactive protein levels in Alzheimer disease. J. Geriatr. Psychiatry Neurol. 23:49-53.
24. Park, S.Y., C.Tournell, R.C.Sinjoanu, and A.Ferreira. 2007. Caspase-3- and calpain-mediated tau cleavage are differentially prevented by estrogen and testosterone in beta-amyloid-treated hippocampal neurons. Neuroscience 144:119-127.
25. Ray, S., M.Britschgi, C.Herbert, Y.Takeda-Uchimura, A.Boxer, K.Blennow, L.F.Friedman, D.R.Galasko, M.Jutel, A.Karydas, J.A.Kaye, J.Leszek, B.L.Miller, L.Minthon, J.F.Quinn, G.D.Rabinovici, W.H.Robinson, M.N.Sabbagh, Y.T.So, D.L.Sparks, M.Tabaton, J.Tinklenberg, J.A.Yesavage, R.Tibshirani, and T.Wyss-Coray. 2007. Classification and prediction of clinical Alzheimer's diagnosis based on plasma signaling proteins. Nat. Med. 13:1359-1362.
26. Reifert, J., D.Hartung-Cranston, and S.C.Feinstein. 2011. Amyloid {beta}-Mediated Cell Death of Cultured Hippocampal Neurons Reveals Extensive Tau Fragmentation without Increased Full-length Tau Phosphorylation. J. Biol. Chem. 286:20797-20811.
27. Rissman, R.A., W.W.Poon, M.Blurton-Jones, S.Oddo, R.Torp, M.P.Vitek, F.M.LaFerla, T.T.Rohn, and C.W.Cotman. 2004. Caspase-cleavage of tau is an early event in Alzheimer disease tangle pathology. J. Clin. Invest 114:121-130.
28. Rosen, W.G., R.C.Mohs, and K.L.Davis. 1984. A new rating scale for Alzheimer's disease. Am. J. Psychiatry 141:1356-1364.
29. Sorensen,M.G., Y.Wang, M.Pajak, K.Duffin, A.C.Bay-Jensen, N.Barascuk, M.A.Karsdal, and K.Henriksen. Utilization of a protease-generated fragment of tau as a biomarker of Alzheimer's disease. 8[4], Suppl. P240. 2012. Alzheimer's & Dementia.
30. Toledo, J.B., L.M.Shaw, and J.Q.Trojanowski. 2013. Plasma amyloid beta measurements - a desired but elusive Alzheimer's disease biomarker. Alzheimers. Res. Ther. 5:8.
31. Wang,Y., M.G.Sorensen, Q.Zheng, C.Zhang, Karsdal M.A., and K.Henriksen. Will Post-translational Modifications of Brain Proteins Provide Novel Serological Markers for Dementias? Int J Alz Disease.
32. Yaffe, K., A.Weston, N.R.Graff-Radford, S.Satterfield, E.M.Simonsick, S.G.Younkin, L.H.Younkin, L.Kuller, H.N.Ayonayon, J.Ding, and T.B.Harris. 2011. Association of plasma beta-amyloid level and cognitive reserve with subsequent cognitive decline. JAMA 305:261-266.
33. Zetterberg, H. 2008. Biomarkers reflecting different facets of Alzheimer's disease. Eur. J. Neurol. 15:1143-1144.
34. Mette Sorensen et al., "Utilization of a protease-generated fragment of tau as a biomarker of Alzheimer's disease", Alzheimer's & Dementia, (2012), vol. 8, no. 4, page P240
35. Basurto-Islas, G et al., "Accumulation of aspartic acid 421- and glutamic acid 391-cleaved tau in neurofibrillary tangles correlates with progression in Alzheimer disease.", Journal Of Neuropathology And Experimental Neurology (2008), vol. 67, no. 5, pages 470 - 483

## Claims

1. A method of assay of a sample of body fluid which comprises, in either order:
a) conducting a first immunoassay of the sample to determine a first level of binding therein of an antibody having specificity for a C-terminal epitope present at the terminus of the amino acid sequence -SSTGSIDMVD(SEQ ID NO:1), and
b) conducting a second immunoassay of the sample to determine a second level of binding therein of an immunological binding partner having specificity for an N-terminal epitope present at the terminus of the amino acid sequence TPRGAAPPGQ- (SEQ ID NO:2),
and
c) calculating an index expressing the information content of a ratio between said second level and said first level.

2. A method as claimed in claim 1, wherein said index is calculated to express the information content of a ratio (Tau-A'/Tau-C'), wherein Tau-A' is said first level and Tau-C' is said second level, each being expressed as a coefficient of the Normal Reference Mean of the measurement.

3. A method as claimed in claim 1 or claim 2, wherein said sample is a sample of blood, serum, plasma, CSF, saliva, sputum, tissues, extracts of tissue, BALF fluid.

4. A method for determining which patients should be enrolled in a clinical trial of a medicament for treatment or prevention of Alzheimer's disease comprising for each candidate patient conducting a method of assay as claimed in any one of claims 1 to 3, and selecting for enrolment patients having an index predictive of a change in ADAS-Cog11 of at least three points over 52 weeks.

5. A method as claimed in claim 4, wherein patients are selected having an index predictive of a change in ADAS-Cog11 of at least three points over 40 weeks.

6. A method as claimed in claim 4, wherein patients are selected having an index predictive of a change in ADAS-Cog11 of at least three points over 28 weeks.

7. A method as claimed in any one of claims 4 to 6, wherein patients are selected having an index predictive of a change in ADAS-Cog11 of at least six points over the stated number of weeks.

8. A method for determining which patients should be enrolled in a clinical trial of a medicament for treatment or prevention of Alzheimer's disease comprising for each candidate patient conducting a method of assay as claimed in claim 2, and selecting for enrolment patients having an index corresponding to a (Tau-A'/Tau-C') ratio of greater than 1.5.

9. A method as claimed in claim 8, comprising selecting for enrolment patients having a said index corresponding to a (Tau-A'/Tau-C') ratio of greater than 2.0.

10. A method as claimed in claim 8, wherein the selected patients have a (Tau-A'/Tau-C') ratio of greater than 2.5 or higher.

11. A method for determining the efficacy of a candidate intervention for treatment or prevention of Alzheimer's disease comprising carrying out an assay as claimed in any one of claims 1 to 3 on a sample obtained from each patient in a first panel of patients and a second panel of patients wherein said intervention has been applied to said first panel of patients and a comparator intervention has been applied to said second panel of patients, and comparing the values for said index obtained for said first panel of patients with values for said index obtained for said second panel of patients, and determining whether the candidate intervention shows a superior effect in modulating said index values in comparison to the comparator intervention.

12. A method for selecting patients for treatment with an intervention for treatment or prevention of Alzheimer's disease comprising conducting a method of assay as claimed in any one of claims 1-3 and selecting patients for treatment who have a resulting index as defined in any one of claims 4 to 11.

## Patentansprüche

1. Verfahren zum Testen einer Probe eines Körperfluids, das in jeglicher Reihenfolge Folgendes umfasst:
a) Durchführen eines ersten Immunassays der Probe, um ein erstes Bindungsniveau eines Antikörpers darin mit einer Spezifität für ein C-terminales Epitop, das am Terminus der Aminosäuresequenz -SSTGSIDMVD (SEQ ID NO:1) vorhanden ist, zu bestimmen, und
b) Durchführen eines zweiten Immunassays der Probe, um ein zweites Bindungsniveau eines immunologischen Bindungspartners darin mit einer Spezifität für ein N-terminales Epitop, das am Terminus der Aminosäuresequenz TPRGAAPPGQ- (SEQ ID NO:2) vorhanden ist, zu bestimmen, und
c) Berechnen eines Index, der den Informationsgehalt eines Verhältnisses zwischen dem ersten Spiegel und dem zweiten Spiegel ausdrückt.

2. Verfahren nach Anspruch 1, wobei der Index berechnet wird, um den Informationsgehalt eines Verhältnisses (Tau-A'/Tau-C') auszudrücken, wobei Tau-A' das erste Niveau ist und Tau-C' das zweite Niveau ist, die jeweils als ein Koeffizient des normalen Referenzmittelwerts der Messung ausgedrückt sind.

3. Verfahren nach Anspruch 1 oder Anspruch 2, wobei die Probe eine Blut-, Serum-, Plasma-, CSF-, Speichel-, Sputum-, Gewebe-, Gewebeextrakt- oder BALF-Fluid-Probe ist.

4. Verfahren zum Bestimmen, welche Patienten in einer klinischen Studie eines Medikaments zur Behandlung oder Vorbeugung von Morbus Alzheimer aufgenommen werden sollten, umfassend das Durchführen eines Verfahrens zum Testen nach einem der Ansprüche 1 bis 3 für jeden Patienten und Auswählen zum Aufnehmen von Patienten mit einem Index, der für eine Veränderung im ADAS-Cog11 von mindestens drei Punkten über 52 Wochen prädiktiv ist.

5. Verfahren nach Anspruch 4, wobei Patienten mit einem Index ausgewählt werden, der für eine Veränderung im ADAS-Cog11 um mindestens drei Punkte über 40 Wochen prädiktiv ist.

6. Verfahren nach Anspruch 4, wobei Patienten mit einem Index ausgewählt werden, der für eine Veränderung im ADAS-Cog11 um mindestens drei Punkte über 28 Wochen prädiktiv ist.

7. Verfahren nach einem der Ansprüche 4 bis 6, wobei Patienten mit einem Index ausgewählt werden, der für eine Veränderung im ADAS-Cog11 um mindestens sechs Punkte über die genannte Anzahl von Wochen prädiktiv ist.

8. Verfahren zum Bestimmen, welche Patienten in einer klinischen Studie eines Medikaments zur Behandlung oder Vorbeugung von Morbus Alzheimer aufgenommen werden sollten, umfassend das Durchführen eines Verfahrens zum Testen nach Anspruch 2 für jeden Patienten und Auswählen zum Aufnehmen von Patienten mit einem Index, der einem (Tau-A'/Tau-C')-Verhältnis größer als 1,5 entspricht.

9. Verfahren nach Anspruch 8, umfassend das Auswählen zum Aufnehmen von Patienten mit diesem Index, der einem (Tau-A'/Tau-C')-Verhältnis größer als 2,0 entspricht.

10. Verfahren nach Anspruch 8, wobei die ausgewählten Patienten mit diesem Index ein (Tau-A'/Tau-C')-Verhältnis größer als 2,5 oder mehr aufweisen.

11. Verfahren zum Bestimmen der Wirksamkeit einer Kandidatenintervention zur Behandlung oder Prävention von Morbus Alzheimer, umfassend das Durchführen eines Tests nach einem der Ansprüche 1 bis 3 an einer Probe, die von jedem Patienten in einer ersten Gruppe von Patienten und einer zweiten Gruppe von Patienten erhalten wurde, wobei die Intervention bei einer ersten Gruppe von Patienten angewandt wurde und eine Vergleichsintervention bei der zweiten Gruppe von Patienten angewandt wurde, und Vergleichen der Werte des für die erste Gruppe von Patienten erhaltenen Index mit den Werten des für die zweite Gruppe von Patienten erhaltenen Index und Bestimmen, ob die Kandidatenintervention eine überlegene Wirkung bei der Modulation dieser Indexwerte verglichen mit der Vergleichsintervention zeigt.

12. Verfahren zum Auswählen von Patienten zur Behandlung mit einer Intervention zur Behandlung oder Vorbeugung von Morbus Alzheimer, umfassend das Durchführen eines Verfahrens zum Testen nach einem der Ansprüche 1 bis 3 und Auswählen von Patienten zur Behandlung, die einen resultierenden Index wie in einem der Ansprüche 4 bis 11 definiert aufweisen.

## Revendications

1. Méthode d'essai d'un échantillon de fluide corporel, qui comprend, dans n'importe quel ordre :
a) conduire un premier immuno-essai de l'échantillon pour déterminer un premier niveau de liaison dans celui-ci d'un anticorps ayant une spécificité pour un épitope C-terminal présent à l'extrémité terminale de la séquence d'acides aminés - SSTGSIDMVD (SEQ ID NO: 1) ; et
b) conduire un second immuno-essai de l'échantillon pour déterminer un second niveau de liaison dans celui-ci d'un partenaire de liaison immunologique ayant une spécificité pour un épitope N-terminal présent à l'extrémité terminale de la séquence d'acides aminés TPRGAAPPGQ - (SEQ ID NO: 2) ; et
c) calculer un indice exprimant le contenu d'information d'un rapport entre ledit second niveau et ledit premier niveau.

2. Méthode selon la revendication 1, dans laquelle ledit indice est calculé pour exprimer le contenu d'information d'un rapport (Tau-A'/Tau-C'), où Tau-A' est ledit premier niveau et Tau-C' est ledit second niveau, chacun étant exprimé en tant que coefficient de la Moyenne de Référence Normale de la mesure.

3. Méthode selon la revendication 1 ou la revendication 2, dans laquelle ledit échantillon est un échantillon de sang, de sérum, de plasma, de liquide céphalorachidien, de salive, d'expectoration, de tissus, d'extraits de tissu, de fluide de lavage broncho-alvéolaire.

4. Méthode pour déterminer quels patients devraient être enrôlés dans un essai clinique d'un médicament pour le traitement ou la prévention de la maladie d'Alzheimer, comprenant, pour chaque patient candidat : conduire une méthode d'essai selon l'une quelconque des revendications 1 à 3, et sélectionner pour l'enrôlement des patients ayant un indice prédictif d'un changement dans ADAS-Cog11 d'au moins trois points en l'espace de 52 semaines.

5. Méthode selon la revendication 4, dans laquelle des patients sont sélectionnés ayant un indice prédictif d'un changement dans ADAS-Cog11 d'au moins trois points en l'espace de 40 semaines.

6. Méthode selon la revendication 4, dans laquelle des patients sont sélectionnés ayant un indice prédictif d'un changement dans ADAS-Cog11 d'au moins trois points en l'espace de 28 semaines.

7. Méthode selon l'une quelconque des revendications 4 à 6, dans laquelle des patients sont sélectionnés ayant un indice prédictif d'un changement dans ADAS-Cog11 d'au moins six points en l'espace du nombre indiqué de semaines.

8. Méthode pour déterminer quels patients devraient être enrôlés dans un essai clinique d'un médicament pour le traitement ou la prévention de la maladie d'Alzheimer, comprenant, pour chaque patient candidat : conduire une méthode d'essai selon la revendication 2, et sélectionner pour l'enrôlement des patients ayant un indice correspondant à un rapport (Tau-A'/Tau-C') de plus de 1,5.

9. Méthode selon la revendication 8, comprenant la sélection pour l'enrôlement de patients ayant un indice précité correspondant à un rapport (Tau-A'/Tau-C') de plus de 2,0.

10. Méthode selon la revendication 8, dans laquelle les patients sélectionnés ont un rapport (Tau-A'/Tau-C') de plus de 2,5 ou plus.

11. Méthode pour déterminer l'efficacité d'une intervention de candidats pour le traitement ou la prévention de la maladie d'Alzheimer, comprenant la réalisation d'un essai selon l'une quelconque des revendications 1 à 3 sur un échantillon obtenu à partir de chaque patient dans un premier panel de patients et un second panel de patients, dans laquelle ladite intervention a été appliquée audit premier panel de patients et une intervention de comparateur a été appliquée audit second panel de patients, et comparer les valeurs pour ledit indice obtenues pour ledit premier panel de patients avec des valeurs pour ledit indice obtenues pour ledit second panel de patients, et déterminer si ou non l'intervention de candidat présente un effet supérieur dans la modulation desdites valeurs d'indice par comparaison avec l'intervention de comparateur.

12. Méthode pour sélectionner des patients pour le traitement avec une intervention pour le traitement ou la prévention de la maladie d'Alzheimer, comprenant : conduire une méthode d'essai selon l'une quelconque des revendications 1 à 3 et sélectionner des patients pour le traitement qui ont un indice résultant tel que défini à l'une quelconque des revendications 4 à 11.
